# EUROPEAN PATENT APPLICATION

(11) **EP 3 915 642 A1**
(43) Date of publication of application: **01.12.2021**
(21) Application number: 19888941.2
(22) Date of filing: 29.11.2019
(51) Int. Cl.: A61Q 1/02, A61Q 17/04, A61Q 19/00, A61Q 19/02, C08L 101/08, A61Q 5/00, A61K 8/06, A61K 8/39, A61K 8/42, A61K 8/81

(54) **NOVEL COMPOSITE BODY AND EMULSION COMPOSITION**

(30) Priority: 30.11.2018 JP 2018225787
(71) Applicant: Kokyu Alcohol Kogyo Co., Ltd., Narita-shi Chiba 287-0225 (JP)
(72) Inventor: OMURA, Takayuki, Narita-shi, Chiba 287-0225 (JP); HANADA, Naomi, Narita-shi, Chiba 287-0225 (JP)
(74) Representative: Cabinet Nuss
(86) International application number: PCT/JP2019/046730
(87) International publication number: WO 2020/111219

(57) **Abstract**

The purpose of the present invention is to provide a novel emulsion composition which is able to be used as an emulsifying agent. Another purpose of the present invention is to provide a novel composite body which is used for emulsification. An emulsion composition according to the present invention contains a carboxy group-containing polymer, an amide alcohol, and a polyglycerol fatty acid ester having one or more hydroxy groups. A composite body according to the present invention, which is used for O/W emulsification, is obtained by bonding a carboxy group-containing polymer, an amide alcohol, and a polyglycerol fatty acid ester having one or more hydroxy groups with each other.

## Description

### [Technical Field]

The present invention relates to a novel complex having emulsifying properties and an emulsion composition.

### [Background Art]

As an emulsion composition for cosmetic materials, conventionally, cream, milky lotion, beauty essence, etc. in which various oily and aqueous raw materials are emulsified are widely used. In producing such an emulsion composition, in addition to processing materials under strictly set various conditions, it has been necessary to use a surfactant in order to ensure temporal stability of the emulsion composition. In recent years, however, even higher safety is expected for cosmetic materials, and from this viewpoint, presence of a surfactant may be a problem.

To solve such a problem, as an emulsion composition without using a surfactant, an emulsion composition which is characterized by comprising, for example, one or more kinds of oils selected from the group consisting of fatty acids and higher alcohols, one or more kinds of polymers soluble to said oils, and one or more kinds of compounds selected from the group consisting of inorganic salts, organic acid salts, amino acids and salts thereof, and not comprising a surfactant, has been proposed (Patent Document 1). However, PVP (polyvinylpyrrolidone) used as one or more kinds of oil-soluble polymers tends to exhibit stickiness, and there is a problem in feeling of use.

An emulsifier capable of providing an emulsion without using a surfactant, and having good feeling of use, has not yet been obtained.

In addition, a secondary amide including an amide alcohol has been studied as an agent imparting moisture resistance, and in a composition using this, formulation wherein a secondary amide is used together with a hydrophilic acrylic polymer (a carboxy group-containing polymer) as a thickener has been prepared (Patent Document 2). However, the secondary amide used in the emulsion prepared in this document does not contain an amide alcohol, and in the preparation method described in this document, no complex is formed, and the use of a common emulsifier (surfactant) is required in order to prepare an emulsion composition.

### [Citation List]

### [Patent Document]

[Patent Document 1] JP A No. 2003-252723
[Patent Document 2] JP A No. H1-502116

### [Disclosure of Invention]

### [Problems to Be Solved by the Invention]

In view of the above-described problems in the prior art, an object of the present invention is to provide an agent and a complex exhibiting an emulsifying power. It is a further object of the present invention to provide an emulsion utilizing such an emulsifying power. Another object of the present invention is to provide an emulsifier capable of obtaining an emulsion excellent in stability, and a stable emulsion. Moreover, a further object of the present invention is to provide an emulsifier capable of obtaining an emulsion excellent in feeling of use on the skin and hair, and an emulsion having excellent feeling of use.

### [Means for Solving the Problems]

During extensive research to solve the above problems, the present inventors have discovered that emulsifying properties can be improved by using an amide alcohol, a polymer containing a carboxy group, and further a polyglycerol fatty acid ester having a hydroxy group; and as a result of further research, the inventors have completed the present invention.

That is, the present invention relates to the following [1] to [9].
[1] An emulsion composition comprising a carboxy group-containing polymer, an amide alcohol represented by formula (I): 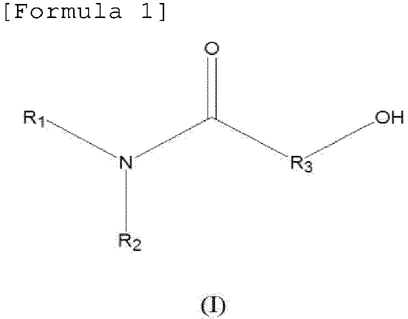 wherein
   R₁ is a C6-C22 hydrocarbon group,
   R₂ is H, or a C6-C22 hydrocarbon group,
   R₃ is a linear or branched C2-C21 hydrocarbon group,
   and a polyglycerol fatty acid ester having one or more hydroxy groups.
[2] The emulsion composition according to [1], wherein the carboxy group-containing polymer has a weight average molecular weight of 500,000 to 3,000,000 and a carboxy group content of 50 to 70%.
[3] The emulsion composition according to [1] or [2], wherein the polyglycerol fatty acid ester having a hydroxy group is represented by formula (II): 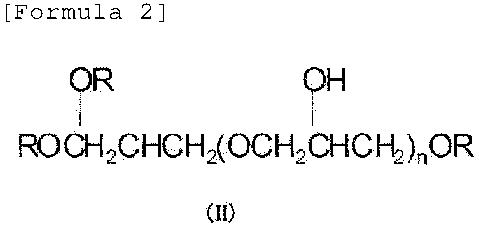 or formula (III): 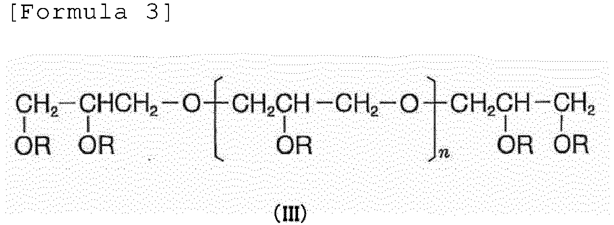 wherein R each independently represents a fatty acid residue or H,
   n is an integer of 2 to 10.
[4] The emulsion composition according to any one of [1] to [3], wherein the polyglycerol fatty acid ester having a hydroxy group has an HLB of 2 to 8.
[5] The emulsion composition according to any one of [1] to [4], further comprising a higher fatty acid.
[6] The emulsion composition according to any one of [1] to [5], which is an O/W type.
[7] The emulsion composition according to any one of [1] to [5], which is a W/O type.
[8] A complex formed by binding a carboxy group-containing polymer, an amide alcohol represented by formula (I): 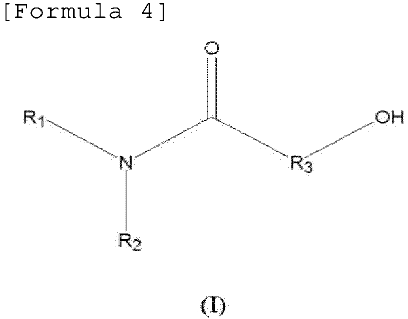 wherein
   R₁ is a C6-C22 hydrocarbon group,
   R₂ is H, or a C6-C22 hydrocarbon group,
   R₃ is a linear or branched C2-C21 hydrocarbon group;
   a polyglycerol fatty acid ester having a hydroxy group, and
   a higher fatty acid.
[9] The complex according to [8], which is used for emulsification.

### [Advantageous Effects of Invention]

The present invention provides an emulsion composition that can be used as an emulsifier and a novel complex that can be used as an emulsifier. The complex of the present invention exhibits high emulsifying power by hydrogen-bonding a polyglycerol fatty acid ester having a hydroxy group with a carboxy group-containing polymer, an amide alcohol and a higher fatty acid, thereby enabling to provide an emulsion having excellent stability. In addition, the present invention can also provide a stable emulsion without using a surfactant. Furthermore, the present invention can provide an emulsion that has an excellent usability and can be used as a cosmetic material.

### [Brief Description of Drawings]

[Fig. 1] Figure 1 is a diagram showing particles of the emulsion when RISOREX PGIS21 is used in the W/O emulsion composition of Example 1.
[Fig. 2] Figure 2 is a diagram showing particles of the emulsion when RISOREX PGIS22 is used in the W/O emulsion composition of Example 1.
[Fig. 3] Figure 3 is a diagram showing particles of the emulsion when RISOREX PGIS23 is used in the W/O emulsion composition of Example 1.
[Fig. 4] Figure 4 is a diagram showing particles of the emulsion when RISOREX PGIS32 is used in the W/O emulsion composition of Example 1.
[Fig. 5] Figure 5 is a diagram showing particles of the emulsion of Comparative Example 1.
[Fig. 6] Figure 6 is a diagram showing particles of the emulsion of Comparative Example 2.
[Fig. 7] Figure 7 is a diagram showing particles of the emulsion of Comparative Example 3.
[Fig. 8] Figure 8 is a diagram showing particles of the emulsion of Comparative Example 4.
[Fig. 9] Figure 9 is a diagram showing particles of the emulsion of Comparative Example 5.
[Fig. 10] Figure 10 is a diagram showing particles of the emulsion when various polyglycerol fatty acid esters are used in Example 2.

### [Embodiments for Carrying out the Invention]

### Ingredient (A): amide alcohol

The amide alcohol used in the present invention is represented by the following formula (I): 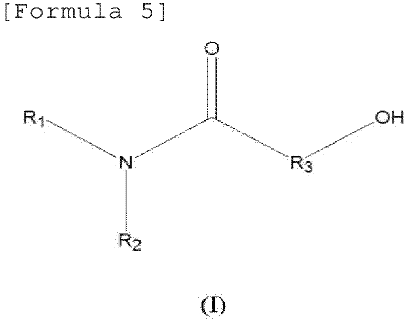 wherein
R₁ is a C6-C22 hydrocarbon group,
R₂ is H, or a C6-C22 hydrocarbon group,
R₃ is a linear or branched C2-C21 hydrocarbon group.

In one embodiment of the present invention, an amide alcohol of formula (I) wherein R₁ is a C10-C22 hydrocarbon group, R₂ is H, and R₃ is a C3-C12 hydrocarbon group is preferred, and an amide alcohol of formula (I) wherein R₁ is a C12-C18 hydrocarbon group, R₂ is H, and R₃ is a C3-C5 hydrocarbon group is particularly preferred.

In another embodiment of the present invention, an amide alcohol of formula (I), wherein R₁ is a linear or branched unsaturated C10-C22 hydrocarbon group; or a cyclic C6-C22 hydrocarbon group; or a benzyl group or phenylethyl group, is preferred.

In a preferred embodiment of the present invention, the amide alcohol of formula (I) has a structure of formulas (I-1) to (1-4) below:

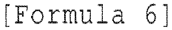

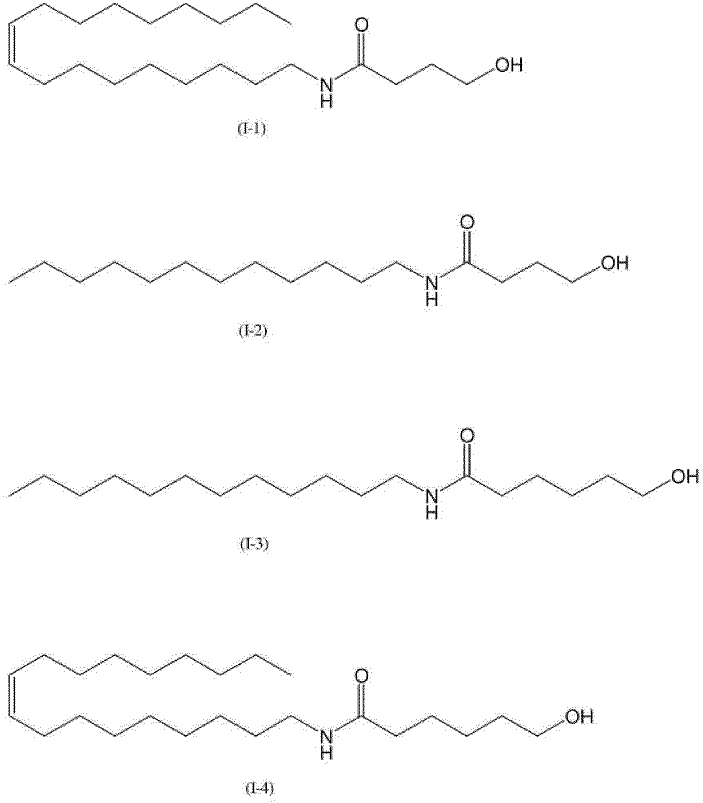

As used herein, the term "hydrocarbon group" may be, unless otherwise specified, saturated or unsaturated, linear, branched or cyclic, or a combination of linear or branched with cyclic and includes, for example, a hydrocarbon group consisting of a linear or branched hydrocarbon moiety and a cyclic hydrocarbon moiety such as benzyl group, phenylethyl group, etc.

That is, the C6-C22 hydrocarbon group in R₁ and R₂ includes a linear, branched or cyclic C6-C22 hydrocarbon group, or a C6-C22 hydrocarbon group consisting of a linear or branched hydrocarbon moiety and a cyclic hydrocarbon moiety, and examples thereof include cyclic groups such as cyclohexyl, decahydronaphthyl, tetrahydrodicyclopentadiene, sterol, phenyl, naphthyl, anthracenyl, etc.; branched alkyl groups such as ethylhexyl, isostearyl, octyldodecyl, etc.; multibranched alkyl groups such as dimethyl, trimethyl, tetramethyl, etc.; linear alkyl groups such as hexyl, octyl, lauryl, myristyl, cetyl, stearyl, aralkyl, behenyl, etc.; and alkenyl groups such as oleyl and elaidyl, etc.

In one embodiment of the invention, R₁ is preferably cyclohexyl, ethylhexyl, octyl, lauryl, myristyl, stearyl, oleyl, benzyl or phenylethyl, with lauryl and oleyl being particularly preferred.

In one embodiment of the present invention, R₂ is preferably H.

The hydrocarbon group in R₃ is a linear or branched C2-C21 hydrocarbon group having no cyclic structure, and examples thereof include alkyl groups such as propyl, butyl, pentyl, hexyl, heptyl, octyl, ethylhexyl, etc., and alkenyl groups such as butylene, pentylene, hexylene, heptylene, etc.

In one embodiment of the present invention, R₃ is preferably propylene, butylene, pentylene or hexylene.

Amide alcohols can be prepared using known synthetic methods. Examples include:
aminolysis reaction of acid chloride and amine (Schotten-Baumann reaction),
aminolysis reaction of anhydrous fatty acid and amine,
aminolysis reaction of methyl ester and amine,
aminolysis reaction of fatty acid and amine,
aminolysis reaction of lactone and amine,
and the like.

Specifically, for example, it can be synthesized by a method described in JP A No. 2016-114276.

### Ingredient (B): carboxy group-containing polymer

The carboxy group-containing polymer used in the present invention is not particularly limited as long as it is a polymer having a carboxy group in the molecule. From the viewpoint of providing appropriate emulsifying ability, typically, those having a weight average molecular weight of 500,000 to 3,000,000 and a carboxy group content in the molecular weight of approximately 50 to 70% are preferred. "Carboxy group content in the molecular weight of 50 to 70%" means that, of the repeating unit parts which are considered to contain carboxy groups in the basic skeleton of the polymer, carboxy groups are present in 50 to 70% of said repeating unit parts.

The carboxy group-containing polymer becomes water-soluble by neutralization with an alkaline substance, and it is generally used as a thickener.

Examples of carboxy group-containing polymer include carboxyvinyl polymer, and alkyl-modified carboxyvinyl polymers such as alkyl acrylate/methacrylate copolymer, etc., acrylic polymers such as alkyl acrylate/alkyl methacrylate polyoxyethylene ester copolymer, alkyl acrylate/alkyl itaconate polyoxyethylene ester copolymer, steareth-10 allyl ether/alkyl acrylate copolymer, etc., and non-acrylic polymers such as methyl vinyl ether/maleic anhydride/decadiene copolymer, etc.

As the carboxy group-containing polymer, carboxyvinyl polymers and alkyl-modified carboxyvinyl polymers are particularly preferred.

A carboxyvinyl polymer, also called carbomer (INCI name: Carbomer), is a polymer having a structure represented by the following formula (II): 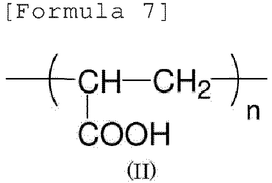 wherein
n is an integer, which is typically from 40 to 100.

Specifically, examples include carboxyvinyl polymers commercially available under the following trade name:
Acritamer 934 (Rita Corporation)
Acritamer 940 (Rita Corporation)
Acritamer 941 (Rita Corporation)
Acritamer 990 (Rita Corporation)
Acritamer 501E (Rita Corporation)
Acritamer 504E (Rita Corporation)
Acritamer 505E (Rita Corporation)
AEC Carbomer 940 (A & E Connock (Perfumery & Cosmetics) Ltd.)
Aqupec HV-501 (Sumitomo Seika Chemicals Co., Ltd.)
Aqupec HV-504 (Sumitomo Seika Chemicals Co., Ltd.)
Aqupec HV-505 (Sumitomo Seika Chemicals Co., Ltd.)
Aqupec HV-501E (Sumitomo Seika Chemicals Co., Ltd.)
Aqupec HV-504E (Sumitomo Seika Chemicals Co., Ltd.)
Aqupec HV-505E (Sumitomo Seika Chemicals Co., Ltd.)
Aqupec HV-801E (Sumitomo Seika Chemicals Co., Ltd.)
Aqupec HV-805E (Sumitomo Seika Chemicals Co., Ltd.)
Aqupec HV-505ED (Sumitomo Seika Chemicals Co., Ltd.)
Aqupec HV-801EG (Sumitomo Seika Chemicals Co., Ltd.)
Aqupec HV-805EG (Sumitomo Seika Chemicals Co., Ltd.)

Carbopol Clear Polymer (Lubrizol Advanced Materials, Inc.)
Carbopol ETD 2050 Polymer (Lubrizol Advanced Materials, Inc.)
Carbopol 934 Polymer (Lubrizol Advanced Materials, Inc.)
Carbopol 940 Polymer (Lubrizol Advanced Materials, Inc.)
Carbopol 941 Polymer (Lubrizol Advanced Materials, Inc.)
Carbopol 980 Polymer (Lubrizol Advanced Materials, Inc.)
Carbopol 981 Polymer (Lubrizol Advanced Materials, Inc.)
Carbopol 2984 Polymer (Lubrizol Advanced Materials, Inc.)
Carbopol 5984 Polymer (Lubrizol Advanced Materials, Inc.)
Carbopol Ultrez 10 Polymer (Lubrizol Advanced Materials, Inc.)
Carbopol Ultrez 30 Polymer (Lubrizol Advanced Materials, Inc.)
CustoPoly J 100 (Custom Ingredients, Inc.)
CustoPoly J 300 (Custom Ingredients, Inc.)
CustoPoly J 400 (Custom Ingredients, Inc.)

Easygel DO (3V Sigma USA Inc.)
Flogel 700 (SNF SAS)
Flogel 1000 (SNF SAS)
Junlon PW-110 (Nihon Junyaku Company, Ltd.)
Junlon PW-111 (Nihon Junyaku Company, Ltd.)
Junlon PW-302S (Nihon Junyaku Company, Ltd.)
Polacril 40 (Lehvoss Italia S.r.l.)
Polygel CA (3V Sigma USA Inc.)
Polygel CB (3V Sigma USA Inc.)
Polygel CS (3V Sigma USA Inc.)
Polygel DV (3V Sigma USA Inc.)
Polygel TG (3V Sigma USA Inc.)
SuperGel CE (Sino Lion USA)
Synthalen K (3V Sigma USA Inc.)
Synthalen L (3V Sigma USA Inc.)
Synthalen M (3V Sigma USA Inc.)
Tego Carbomer 134 (Evonik Nutrition & Care GmbH)
Tego Carbomer 140 (Evonik Nutrition & Care GmbH)
Tego Carbomer 141 (Evonik Nutrition & Care GmbH)
Tego Carbomer 340 FD (Evonik Nutrition & Care GmbH).

The alkyl-modified carboxyvinyl polymer is a copolymer of acrylic acid and/or methacrylic acid with alkyl acrylate and/or alkyl methacrylate thereof.

Specific examples of alkyl-modified carboxyvinyl polymer include acrylates/alkyl acrylates (C10-30)) cross polymer (INCI name: Acrylates/C10-30 Alkyl Acrylates Cross polymer, also called alkyl acrylate/methacrylate copolymer), which is a polymer having a structure represented by the following formula (III): 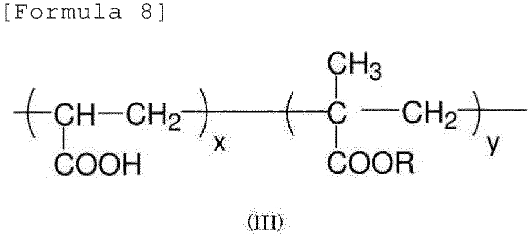 wherein
R is a C10-30 alkyl group,
x and y are integers, each of which can be arbitrarily selected from integers of 1 or more, and typically x + y = 40 to 100, and when y is 2 or more, R may be the same or different.

Specifically, the examples include alkyl-modified carboxyvinyl polymers commercially available under the following trade name:
Acritamer 501ED (Rita Corporation)
Acritamer 505ED (Rita Corporation)
Aqupec HV-701EDR (Sumitomo Seika Chemicals Co., Ltd.)
Aqupec HV-501ER (Sumitomo Seika Chemicals Co., Ltd.)
Aqupec SER W-150C (Sumitomo Seika Chemicals Co., Ltd.)
Aqupec SER W-300C (Sumitomo Seika Chemicals Co., Ltd.)
Carbopol ETD 2020 Polymer (Lubrizol Advanced Materials, Inc.)
Carbopol 1342 Polymer (Lubrizol Advanced Materials, Inc.)
Carbopol 1382 Polymer (Lubrizol Advanced Materials, Inc.)
Carbopol SC 200 (Lubrizol Advanced Materials, Inc.)
Carbopol SC 500 Polymer (Lubrizol Advanced Materials, Inc.)
Carbopol Ultrez 20 Polymer (Lubrizol Advanced Materials, Inc.)
Carbopol Ultrez 21 Polymer (Lubrizol Advanced Materials, Inc.)
Carbopol Xtra-11 Polymer (Lubrizol Advanced Materials, Inc.)
Pemulen EZ-4U Polymeric Emulsifier (Lubrizol Advanced Materials, Inc.)
Pemulen TR-1 Polymer (Lubrizol Advanced Materials, Inc.)
Pemulen TR-2 Polymer (Lubrizol Advanced Materials, Inc.)
Tego Carbomer 341 ER (Evonik Nutrition & Care GmbH)
TEGO Carbomer 750 HD (Evonik Nutrition & Care GmbH).

### Ingredient (C): Polyglycerol fatty acid ester

It is known that a polyglycerol fatty acid ester can be generally used as an emulsifier, a solubilizer or a dispersant; the polyglycerol fatty acid ester used in the present invention is preferably a polyglycerol fatty acid ester having a hydroxy group in the molecule, from the viewpoint that it forms a complex and improves the emulsifying ability.

It is considered that the hydroxy group of the polyglycerol fatty acid ester having a hydroxy group exhibits high emulsifying ability by forming a hydrogen bond with the amide bond portion of the amide alcohol and/or the carbonyl group of the polymer and higher fatty acid.

Therefore, without being bound by any theory, it is considered that in the complex of the present invention, the hydroxy group of the polyglycerol fatty acid ester is hydrogen-bonded to the amide bond moiety of the amide alcohol, the carbonyl group of the polymer and/or the carbonyl group of the higher fatty acid, to form a complex.

The HLB of the polyglycerol fatty acid ester having a hydroxy group of the present invention is not limited from the viewpoint of complex formation, and is preferably 2 to 8, and more preferably 2 to 5.

The polyglycerol fatty acid ester used in the present invention may be those of the formula (II):

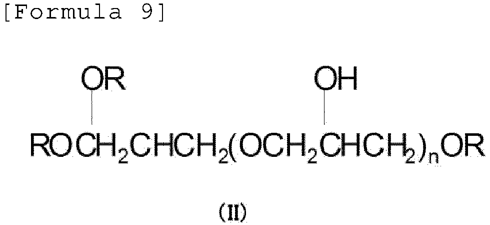

In the formula, R is each independently a fatty acid residue or H, and the fatty acid residue is preferably C10 to C20, more preferably C12 to C18.

Specific examples of polyglycerol fatty acid ester represented by formula (II) include polyglycerol fatty acid esters commercially available under the following trade names:
EMALEX DCCG-3 (Nihon Emulsion Co., Ltd.)
EMALEX DSG-2 (Nihon Emulsion Co., Ltd.)
EMALEX DSG-3 (Nihon Emulsion Co., Ltd.)
EMALEX DSG-6 (Nihon Emulsion Co., Ltd.)
EMALEX TSG-10 (Nihon Emulsion Co., Ltd.)
EMALEX DISG-2 (Nihon Emulsion Co., Ltd.)
EMALEX DISG-2EX (Nihon Emulsion Co., Ltd.)
EMALEX TISG-2 (Nihon Emulsion Co., Ltd.)
EMALEX DISG-3 (Nihon Emulsion Co., Ltd.)
EMALEX DISG-3EX (Nihon Emulsion Co., Ltd.)
EMALEX DISG-6 (Nihon Emulsion Co., Ltd.)
EMALEX TISG-10 (Nihon Emulsion Co., Ltd.)

In addition, they also include polyglycerol fatty acid esters commercially available under the following trade names:
IS-201P (Sakamoto Yakuhin Kogyo Co., Ltd.)
IS-202P (Sakamoto Yakuhin Kogyo Co., Ltd.)
0-201P (Sakamoto Yakuhin Kogyo Co., Ltd.)

Specific examples of polyglycerol fatty acid ester represented by formula (III) include polyglycerol fatty acid esters commercially available under the following trade names:
NIKKOL DGMS (Nikko Chemicals Co., Ltd.)
NIKKOL DGMO-CV (Nikko Chemicals Co., Ltd.)
NIKKOL DGMO-90V (Nikko Chemicals Co., Ltd.)
NIKKOL DGMIS (Nikko Chemicals Co., Ltd.)
NIKKOL Tetraglyn 1-SV (Nikko Chemicals Co., Ltd.)
NIKKOL Hexaglyn 3-SV (Nikko Chemicals Co., Ltd.)
NIKKOL Decaglyn 3-SV (Nikko Chemicals Co., Ltd.)
NIKKOL Decaglyn 3-OV (Nikko Chemicals Co., Ltd.)
NIKKOL Decaglyn 5-SV (Nikko Chemicals Co., Ltd.)
NIKKOL Decaglyn 5-ISV (Nikko Chemicals Co., Ltd.)
NIKKOL Decaglyn 5-OV (Nikko Chemicals Co., Ltd.)

### <Higher fatty acid>

Higher fatty acid used in the present invention is not particularly limited as long as it is an ingredient generally used in cosmetics and the like. Examples include a higher fatty acid represented by the general formula (G):

R₃COOH (G)

wherein R₃ is a linear or branched, saturated or unsaturated hydrocarbon having an average carbon number of 7 to 25 which may have a hydroxy group, and these may be used alone or in combination of two or more kinds.

From the viewpoint of forming a complex suitable for emulsification, a higher fatty acid wherein R₃ in the above general formula (G) is a linear or branched chain having an average carbon number of 11 to 21 is preferred.

Specific examples include saturated fatty acids such as lauric acid, myristic acid, palmitic acid, stearic acid, arachic acid, behenic acid, etc.; unsaturated fatty acids such as 2-palmitoleic acid, petroselinic acid, oleic acid, elaidic acid, ricinoleic acid, linoleic acid, linoelaidic acid, linolenic acid and arachidonic acid, etc.; branched fatty acids such as isostearic acid, etc.; hydroxycarboxylic acids such as 12-hydroxystearic acid, etc. Of these, from the viewpoints of stability and skin irritation, saturated fatty acids having a carbon number of 18 are preferable, and among them, those having branching are preferable, and saturated fatty acids with a carbon number of 18 having methyl branching are more preferable. Examples of commercially available products include isostearic acid (trade name: Isostearic acid EX, HAIMARIC-MKH(R), manufactured by Kokyu Alcohol Kogyo Co., Ltd.), SR PRISORINE 3505 (Croda Japan, K.K.), Isostearic acid, Isostearic acid T (Nissan Chemical Corporation) and the like.

The higher fatty acid is considered to function as a part of the complex in addition to the function as an oil agent in the emulsion.

In one embodiment of the present invention, "complex" means a complex formed from an amide alcohol, a carboxy group-containing polymer, a polyglycerol fatty acid ester, and a higher fatty acid.

The complex can be formed by adding a carboxy group-containing polymer to an oil phase part containing an amide alcohol, a polyglycerol fatty acid ester and a higher fatty acid, or by adding an oil phase part containing an amide alcohol, a polyglycerol fatty acid ester and a higher fatty acid to an aqueous phase part containing a carboxy group-containing polymer, and it can be further neutralized with an alkali to form a complex having a higher emulsifying power.

In one embodiment of the present invention, the complex can be formed by adding a carboxy group-containing polymer to an oil phase part containing an amide alcohol, a polyglycerol fatty acid ester and a higher fatty acid, then neutralized with an alkali.

In another embodiment of the present invention, the complex can be formed by adding an aqueous phase part containing a carboxy group-containing polymer and an alkali to an oil phase part containing an amide alcohol, a polyglycerol fatty acid ester and a higher fatty acid.

The emulsifying ability is considered to be exerted as follows: the amide bond portion of the amide alcohol and the carbonyl group of the polymer and higher fatty acid form a hydrogen bond, and a part of the carboxy group becomes COO‾ to have hydrophilicity.

Therefore, without being bound by any theory, the complex of the present invention is considered to be formed by bonding, more specifically hydrogen bonding the amide bond portion of the amide alcohol with the carbonyl group of the polymer and the carbonyl group of the higher fatty acid.

The emulsion of the present invention is "W/O type" or "O/W type".

In the present invention, the "W/O emulsion" is a water-in-oil emulsion, that is, an emulsion in which an aqueous ingredient is dispersed in a continuous phase containing an oily ingredient.

The W/O emulsion can be prepared by dispersing an aqueous phase containing a carboxy group-containing polymer in an oil phase containing an amide alcohol, and neutralizing with an alkali.

In the present invention, the "O/W emulsion" is an oil-in-water emulsion, that is, an emulsion in which an oily ingredient is dispersed in a continuous phase containing an aqueous ingredient.

The O/W emulsion can be prepared by dispersing an oil phase containing an amide alcohol in an aqueous phase containing a carboxy group-containing polymer and neutralizing with an alkali.

In one embodiment of the present invention, W/O emulsions or O/W emulsions can be used for all purposes, but typically, they can be used for external preparations such as pharmaceuticals, quasi drugs, and cosmetics, etc.

The W/O emulsions or O/W emulsions of the present invention can be used for various forms of products including pharmaceuticals such as external skin preparation comprising a drug; quasi drugs such as medicated cosmetics; skin care cosmetics such as gel lotion, milky lotion, cream, beauty essence, sunscreen, and daytime moisturizer; makeup cosmetics such as foundation, makeup base, eye shadow, mascara, as well as hair care cosmetics such as hair treatment, etc.

### <Oily ingredient>

The oily ingredient used in the W/O emulsion or O/W emulsion of the present invention is not particularly limited as long as it is an ingredient generally used for cosmetics and the like; and examples thereof include oil agents such as animal and vegetable fats and oils, hydrocarbon oils, higher fatty acids, higher alcohols, ester oils, silicone oils, etc., which can be used alone or in combination of two or more kinds.

Examples of animal and vegetable fats and oils or hydrogenated animal and vegetable fats and oils include avocado oil, perilla oil, olive oil, cacao butter, kaya oil, apricot kernel oil, hydrogenated oil, wheat germ oil, sesame oil, rice germ oil, rice bran oil, sugar cane wax, Camellia sinensis leaf oil, safflower oil, shea butter, Chinese tung oil, cinnamon oil, soybean oil, tea seed oil, camellia oil, evening primrose oil, corn oil, rapeseed oil, germ oil, palm oil, palm kernel oil, castor oil, hydrogenated castor oil, sunflower oil, grape oil, jojoba oil, macadamia nut oil, beeswax, cottonseed oil, cotton wax, Japan wax, montan wax, coconut oil, hardened coconut oil, peanut oil, lanolin, liquid lanolin, reduced lanolin, lanolin alcohol, hard lanolin, lanolin acetate, lanolin fatty acid isopropyl ester, hexyl laurate, etc.

Examples of hydrocarbon oils include ozokerite, squalane, squalene, ceresin, paraffin, isoparaffin, paraffin wax, liquid paraffin (mineral oil), pristane, polyisobutylene, polyisobutene, hydrogenated polyisobutene, microcrystalline wax, polyethylene wax, Vaseline, etc.

Examples of higher fatty acids include lauric acid, myristic acid, palmitic acid, stearic acid, isostearic acid, oleic acid, behenic acid, undecylenic acid, oleic acid, linoleic acid, linolenic acid, arachidonic acid, eicosapentaenoic acid (EPA), docosahexaenoic acid (DHA), etc.

Examples of higher alcohols include myristyl alcohol, cetanol, stearyl alcohol, arachidyl alcohol, behenyl alcohol, hydrogenated rapeseed oil alcohol, etc.

Examples of ester oils include, as monoester, isononanoic acid esters such as isononyl isononanoate, isodecyl isononanoate, isotridecyl isononanoate, tricyclodecanemethyl isononanoate, ethylhexyl isononanoate, etc., 2-ethylhexanoic acids such as cetyl ethylhexanoate, hexyldecyl ethylhexanoate, etc., myristic acid esters such as isopropyl myristate, isocetyl myristate, octyldodecyl myristate, etc., isostearic acid esters such as ethyl isostearate, isopropyl isostearate, hexyldecyl isostearate, isostearyl isostearate, cholesteryl isostearate, phytosteryl isostearate, etc., lactic acid esters such as isostearyl lactate, octyldodecyl lactate, etc., oleic acid esters such as oleyl oleate, phytosteryl oleate, octyldodecyl oleate, etc., neopentanoic acid esters such as isodecyl neopentanoate, isostearyl neopentanoate, etc., palmitic acid esters such as isopropyl palmitate, ethylhexyl palmitate, etc., and others such as octyldodecyl neodecanoate, octyldodecyl ricinoleate, oleyl erucate, octyldodecyl erucate, isopropyl lauroyl sarcosinate, etc.

Examples of diester oils include diisobutyl adipate, diisopropyl adipate, diethylhexyl succinate, neopentyl glycol diisononanoate, neopentyl glycol diethylhexanoate, neopentyl glycol dicaprate, diisostearyl malate, diisopropyl dilinoleate, ethylene glycol dioctanoate, octyldodecyl stearoyl oxystearate, diisopropyl sebacate, di(cholesteryl/octyldodecyl) lauroyl glutamate, di(phytosteryl/octyldodecyl) lauroyl glutamate, etc.

Examples of triester oils include triethylhexanoin, trimethylolpropane triethylhexanoate, glyceryl tri(caprylate/caprate), triisostearin, trimethylolpropane triisostearate, etc.

Examples of tetraester oils include pentaerythrityl tetraethylhexanoate, pentaerythrityl tetraisostearate, etc.

Examples of polyester oils include polyglyceryl fatty acid esters such as polyglyceryl-2 isostearate, polyglyceryl-2 diisostearate, polyglyceryl-2 triisostearate, polyglyceryl-2 tetraisostearate, etc.

Examples of highly viscous ester oils include hydrogenated castor oil isostearate, hydrogenated castor oil dimer dilinoleate, (polyglyceryl-2 isostearate/dimer dilinoleate) copolymer, (phytosteryl/isostearyl/cetyl/stearyl/behenyl) dimer dilinoleate, dimer dilinoleyl bis(phytosteryl/behenyl/isostearyl) dimer dilinoleate, di(isostearyl/phytosteryl) dimer dilinoleate, dimer dilinoleyl hydrogenated rosin condensate, dimer dilinoleyl diisostearate, dimer dilinoleyl dimer dilinoleate, di(cholesteryl/behenyl/octyldodecyl) lauroyl glutamate, di(octyldodecyl/phytosteryl/behenyl) lauroyl glutamate, myristoyl methyl alanine (phytosteryl/decyltetradecyl), etc.

Examples of silicone oils include dimethylpolysiloxane, methyl phenyl polysiloxane, alkyl-modified organopolysiloxane, terminal modified organopolysiloxane, fluorine-modified organopolysiloxane, amodimethicone, amino-modified organopolysiloxane, volatile silicone, alkyl dimethicone, cyclopentasiloxane, etc.

In one embodiment of the present invention, the blending amount of the oily ingredient in the W/O emulsion or O/W emulsion is not particularly limited; however, in the case of W/O emulsion, from the viewpoint of usability and stability, it may be 30.0 mass% to 80.0 mass%, preferably 40.0 to 70.0 mass%, and more preferably 50.0 to 60.0 mass%. In contrast, in the case of O/W emulsion, from the viewpoint of usability, it may be 1.0 mass% to 40.0 mass%, preferably 3.0 to 30.0 mass%, and more preferably 5.0 to 20.0 mass%.

In the present invention, from the viewpoint of providing an emulsion that exhibits stability for a longer period of time, it is preferable that the ratio of nonpolar oils such as hydrocarbon oil and silicone oil in the oily ingredient is high.

From the viewpoint of obtaining a long-term stable emulsion without using a surfactant, it is preferable that the mass ratio of nonpolar oil/(nonpolar oil + polar oil) exceeds 0.5.

### <Aqueous ingredient>

Aqueous ingredients used in the W/O emulsion or O/W emulsion of the present invention are not particularly limited as long as they are ingredients generally used in cosmetics and the like; and examples thereof include water such as purified water, ion exchanged water, etc.; and lower alcohols such as BG (1,3-butylene glycol), PG (propylene glycol), glycerin, ethanol, etc., and these can be used alone or in combination of two or more kinds.

In one embodiment of the present invention, the blending amount of the aqueous ingredient in the W/O emulsion is not particularly limited; from the viewpoint of usability and stability, it may be 10.0 to 60.0 mass%, preferably 15.0 to 60.0 mass%, and more preferably 20.0 to 50.0 mass%. In one embodiment of the present invention, the blending amount of the aqueous ingredient in the O/W emulsion is not particularly limited; from the viewpoint of usability and stability, it may be 50.0 to 90.0 mass%, preferably 55.0 to 85.0 mass%, and more preferably 60.0 to 80.0 mass%.

### <Neutralizing agent>

A neutralizing agent used for preparing the W/O emulsion or O/W emulsion of the present invention is not particularly limited as long as it is an alkaline ingredient generally used in cosmetics and the like, and examples thereof include potassium hydroxide, triethanolamine, sodium hydroxide, basic amino acids such as L-arginine and L-lysine, 2-amino-2-methyl-1-propanol, etc., and these can be used alone or in combination of two or more kinds.

The blending amount of a neutralizing agent can be appropriately selected depending on the type of the neutralizing agent and the composition of the whole emulsion, and it is typically about 0.01 to 1.0 mass%.

In addition, the neutralizing agent may be an active ingredient such as tranexamic acid, carnosine, etc.

### <Surfactant>

In the present specification, the term "surfactant" means a compound having both a hydrophilic group and a hydrophobic group in one molecule, and a surfactant may be appropriately added as necessary to W/O emulsions or O/W emulsions.

In one embodiment of the present invention, because the complex of the present invention has an emulsifying ability, preferably W/O emulsions or O/W emulsions are substantially free of surfactant. This makes it possible to provide an emulsifier with less irritation.

Here, "substantially free of" means that a surfactant is not contained in an amount sufficient for the emulsification of the whole W/O emulsion or O/W emulsion. In addition, in the present invention, "substantially free of surfactant" means that it comprises no surfactant at all or comprises a surfactant in an amount that is not emulsified. The amount that is not emulsified can be appropriately determined by a person skilled in the art according to the compositional ratio, for example, in one embodiment it is less than 2.0 mass%, in another embodiment it is less than 0.2 mass%, or less than 0.02 mass%.

In a particular embodiment of the invention, the conditioning composition is a W/O emulsion composition or an O/W emulsion composition that is substantially free of surfactant such as cationic surfactant.

### <Other ingredients>

The emulsion of the present invention may comprise any ingredients used in external preparations such as cosmetics, etc.

Examples of these additional ingredients include ultraviolet absorbers such as ethylhexyl methoxycinnamate, diethylamino hydroxybenzoyl hexyl benzoate, etc.; thickeners and gelling agents such as dextrin palmitate, xanthan gum, etc.; quality maintaining ingredients such as antioxidant, preservative, etc.; skin softeners (emollients); medicinal ingredients and active ingredients such as whitening agent, anti-wrinkle agent, antioxidative agent, etc.; perfumes, coloring agents such as pigment and dyestuff, and the like.

In one embodiment of the present invention, in order to improve stability of the W/O emulsion, the outer oil layer is oil-gelled with an oil gelling agent.

As an ingredient used for oil-gelling, an organically modified clay mineral and/or a polymer ester of sugar and fatty acid can be used.

The organically modified clay mineral and the polymer ester of sugar and fatty acid used in the preparation of the W/O emulsions of the present invention are not particularly limited as long as they are ingredients generally used in cosmetics and the like.

Examples of organically modified clay mineral include water-swellable clay minerals (for example, montmorillonite, saponite, hectorite, bentonite, etc.) in which a convertible cation interposed between crystal layers is replaced with an organic polar compound or an organic cation (for example, a quaternary ammonium salt-type cationic surfactant).

In the present invention, organically modified clay minerals which are commercially available under the trade names of, for example, BENTONE 27V (stearalkonium hectorite), BENTONE 27VCG (stearalkonium hectorite), BENTONE 38V (disteardimonium hectorite), BENTONE 38VCG (disteardimonium hectorite), etc. from Elementis Specialties Inc. can be used.

In addition, organically modified clay minerals are also commercially available as a premixed product dissolved in silicone oil, ester oil and/or other oil agent; in the present invention, such a premix may be used, and for example, it is commercially available from Elementis Specialties Inc. under the following trade names:
BENTONE GEL 1002V (cyclopentasiloxane, disteardimonium hectorite, propylene carbonate), BENTONE GEL ABO V (Crambe abyssinica seed oil, stearalkonium hectorite, propylene carbonate), BENTONE GEL CAO V (castor oil, stearalkonium hectorite, propylene carbonate), BENTONE GEL EUG V (octyldodecanol, disteardimonium hectorite, propylene carbonate), BENTONE GTCC V (glyceryl tri(caprylate/caprate), stearalkonium hectorite, propylene carbonate), BENTONE HSO V (glyceryl tri(caprylate/caprate), stearalkonium hectorite, propylene carbonate), BENTONE IHD V (isohexadecane, disteardimonium hectorite, propylene carbonate), BENTONE IPM V (isopropyl myristate, stearalkonium hectorite, propylene carbonate), BENTONE ISD V (isododecane, disteardimonium hectorite, propylene carbonate), BENTONE LOI V (liquid lanolin, isopropyl palmitate, stearalkonium hectorite, propylene carbonate), BENTONE MSO(V) (meadowfoam oil, disteardimonium hectorite, propylene carbonate), BENTONE NGD V (neopentyl glycol diheptanoate, disteardimonium hectorite, propylene carbonate), BENTONE OLV V (olive fruit oil, stearalkonium hectorite, propylene carbonate), BENTONE OMS V ((C11, 12) isoparaffin, disteardimonium hectorite, denatured alcohol), BENTONE PTIS V (pentaerythrityl tetraisostearate, disteardimonium hectorite, propylene carbonate), BENTONE SS71V (petroleum volatiles, disteardimonium hectorite, propylene carbonate), BENTONE TMF V (methyl trimethicone, disteardimonium hectorite, triethyl citrate), BENTONE TNV ((alkyl (C12-15) benzoate, stearalkonium hectorite, propylene carbonate), BENTONE VS-5V(V) (cyclopentasiloxane, disteardimonium hectorite, denatured alcohol), BENTONE VS-5PC V(HV) (cyclopentasiloxane, disteardimonium hectorite, propylene carbonate).

The blending amount of the organically modified clay mineral in the W/O emulsion or O/W emulsion can be appropriately adjusted depending on the kind and amount of the oily ingredient and the viscosity required for the emulsion, and is not particularly limited; in the case of W/O emulsions, it may be 0.5 to 8.0 mass%, preferably 0.7 to 6.0 mass%, and more preferably 1.0 to 5.0 mass%. In contrast, in the case of O/W emulsions, it may be 1.0 mass% or less.

Examples of polymer esters with sugar and fatty acid include, but are not limited to, those commercially available under the following trade names by Chiba Flour Milling Co. Ltd. :
Rheopearl KL (dextrin palmitate),
Rheopearl TL2 (dextrin palmitate),
Rheopearl MKL2 (dextrin myristate).

The amount of the polymer ester with sugar and fatty acid contained in the W/O emulsion may be 0.5 to 8.0 mass%, preferably 0.7 to 6.0 mass%, and more preferably 1.0 to 5.0 mass%. In contrast, in the case of O/W emulsions, it may be 1.0 mass% or less.

In addition, in one embodiment of the present invention, by blending a polyhydric alcohol such as pentylene glycol, octylene glycol etc., emulsifying properties as a W/O emulsion or an O/W emulsion can be further improved.

Without being bound by any theory, it is considered that improvement of emulsifying properties by blending a polyhydric alcohol is achieved by the formation of a complex by hydrogen bonding of a hydroxy group in the molecule of the polyhydric alcohol and a carbonyl group of the amide bond in the amide alcohol molecule.

Accordingly, the present invention also provides a complex formed from an amide alcohol, a carboxy group-containing polymer, a higher fatty acid and a polyhydric alcohol.

It is to be noted that pentylene glycol has a moisturizing action and an antibacterial action, and it is a preferable ingredient for using emulsion as an external preparation such as cosmetics.

Specifically, pentylene glycol is commercially available under the following trade names: Diol PD (Kokyu Alcohol Kogyo Co., Ltd.) and Diol PD-V (Kokyu Alcohol Kogyo Co., Ltd.).

Specifically, octylene glycol is commercially available under the following trade name: OD-eight (Kankohsha Co., Ltd.) .

The blending amount of the ingredient (A) in the W/O emulsion can be appropriately selected depending on the type and amount of the oil agent to be used and the viscosity required, etc., and it is typically 0.1 to 15. 0 mass%, preferably 0.5 to 10.0% mass%, and more preferably 1.0 to 8.0 mass%.

The blending amount of the ingredient (B) in the W/O emulsion can be appropriately selected depending on the type and amount of the oil agent to be used, the viscosity required for the emulsion composition, etc. Typically, it is 0.01 to 5.0 mass%, preferably 0.05 to 3.0 mass%, and more preferably 0.1 to 2.0 mass%.

By using the above blending amounts, an amount sufficient to obtain an emulsion composition having good water-in-oil type usability and stability of the complex consisting of ingredient A and ingredient B in the present invention is realized.

The blending amount of the ingredient (A) in the O/W emulsion can be appropriately selected depending on the type and amount of the oil agent to be used and the viscosity required, etc., and it is typically 0.1 to 15.0 mass%, preferably 0.5 to 10.0 mass%, and more preferably 1.0 to 8.0 mass%.

The blending amount of the ingredient (B) in the O/W emulsion can be appropriately selected depending on the type and amount of the oil agent to be used and the viscosity required for the emulsion composition, etc. Typically, it is 0.01 to 5.0 mass%, preferably 0.05 to 3.0 mass%, and more preferably 0.1 to 2.0 mass%.

By using the above blending amounts, an amount sufficient to obtain an emulsion composition having good water-in-oil type usability and stability of the complex consisting of ingredient A and ingredient B in the present invention is realized.

In the W/O emulsion composition, the ratio of wt% of amide alcohol (ingredient (A)) and carboxy group-containing polymer (ingredient (B)) in the emulsion composition is preferably amide alcohol/carboxy group-containing polymer = 10 to 110, more preferably 15 to 100, still more preferably 20 to 80.

By using these ratios, the balance of hydrophilicity and hydrophobicity becomes suitable for W/O emulsions, and it is possible to provide an emulsion with high stability having fine emulsion particles.

In the O/W emulsion composition, the ratio of wt% of amide alcohol (ingredient (A)) and carboxy group-containing polymer (ingredient (B)) in the emulsion composition is preferably amide alcohol/carboxy group-containing polymer = 1 to 30, more preferably 5 to 20, and even more preferably 7 to 15.

By using these ratios, the balance of hydrophilicity and hydrophobicity becomes suitable for O/W emulsions, and it is possible to provide an emulsion with high stability having fine emulsion particles.

Furthermore, the blending amount of the higher fatty acid in the W/O emulsion or O/W emulsion can be appropriately selected depending on the type and amount of the oil agent to be used, the viscosity required, etc., and it is typically 0.1 to 7.0 mass%, and more preferably 0.5 to 5.0 mass%.

By using the above blending amounts, the amounts suitable for obtaining an emulsion composition having good usability and stability of the water-in-oil type of complex consisting of amide alcohol, carboxy group-containing polymer and higher fatty acid in the present invention are realized.

The pH of the W/O emulsion or O/W emulsion of the present invention can be appropriately selected, and it is preferably about pH 5.0 to 10.0, more preferably about pH 5.0 to 8.0. Without being bound by any theory, it is considered that, by setting the pH within this range, the ingredient A and the ingredient B (and the higher fatty acid) are moderately hydrogen bonded to form a complex, and the dissociation of the carboxy group becomes moderate, leading to excellent emulsifying ability.

Viscosity of the emulsion of the present invention can be appropriately selected depending on the characteristics of objective products.

In one embodiment of the present invention, from the viewpoint of obtaining a creamy emulsion, it is preferable to have a viscosity higher than 10,000 mPa·s, and from the viewpoint of obtaining an emulsion in the form of milky lotion, it is preferable to have a viscosity of about 300 to 100,000 mPa·s.

In one embodiment of the present invention, from the viewpoint of using emulsion as a cosmetic material, the viscosity of the emulsion can be appropriately selected within the range of 300 to 1,000,000 mPa·s.

The present invention also provides an agent used for emulsification, containing an amide alcohol, a carboxy group-containing polymer, or a higher fatty acid. Such an agent can be used in the production of emulsions.

Hereinafter, the present invention will be described in more detail based on examples and comparative examples; however, the present invention is not limited to these examples, and various modifications can be made without departing from the technical idea of the present invention. In the present specification, unless otherwise specified, % means mass%.

Hereinafter, the particle size and the median diameter (µm) when the polyglycerol fatty acid ester of the present invention was added were measured. Each evaluation was performed by the following method.

### 1. Measurement of emulsion particle size by microscopic observation

Using "BX-51" manufactured by Olympus Corporation, observation was carried out at 400 times magnification, and emulsion particle size of emulsions after neutralization was evaluated. Regarding the notation of the emulsion particle size, "~5(15)" means that it is on average 5 µm or less, but there are particles with a diameter of about 15 µm scattered in some places.

### 2. Median diameter

Median diameter is a diameter of the central particle when particles are arranged in the order from the smallest to the largest, that is, the diameter of the particle which is positioned at 50% of the integrated value from the smallest particle. In this specification, measurement was performed using a laser diffraction particle size analyzer "SALD-2300" (Shimadzu Corporation).

### [Examples]

A. W/O emulsion composition

### (1) Example of W/O emulsion composition

[Example 1] The particle size and median diameter of the W/O emulsion composition according to the following formulation were measured.

**[Table 1]**

| Formulation of W/O emulsion composition | |
|---|---|
| Ingredient name | Blending amount (%) |
| Ion exchanged water | 35. 5 |
| Glycerin | 5.0 |
| 1,3-Butylene glycol | 3. 0 |
| PEMULEN TR-2 | 0. 2 |
| SquaIane (Trade name: Olive squalane, Kokyu Alcohol Kogyo Co., Ltd.) | 36. 1 |
| **Polyglycerol fatty acid ester** | 2.0 |
| Amide alcohol OLH | 6. 0 |
| Isostearic acid | 5. 9 |
| BENTONE GEL ISD V | 3. 5 |
| Rheopearl KL2 | 1. 5 |
| Rheopearl WX | 1.0 |
| Potassium hydroxide | 0.25 |
| Total | 100.00 |

Here, each raw material used in the example is as follows: Polyglycerol fatty acid ester: an ester represented by formula (II), specifically as follows:
▪ RISOREX PGIS21 (polyglyceryl-2 isostearate, HLB value 5.0)
▪ RISOREX PGIS22 (polyglyceryl-2 diisostearate, HLB value 3.0)
▪ RISOREX PGIS23 (polyglyceryl-2 triisostearate, HLB value 2.6)
▪ RISOREX PGIS32 (polyglyceryl-3 diisostearate, HLB value 4.5) PEMULEN TR-2: trade name (acrylate/alkyl acrylate(C10-30)) cross polymer, product of Lubrizol Advanced Materials, Inc. Amido alcohol OLH: amide alcohol represented by formula (1-4) (Kokyu Alcohol Kogyo Co, Ltd.)

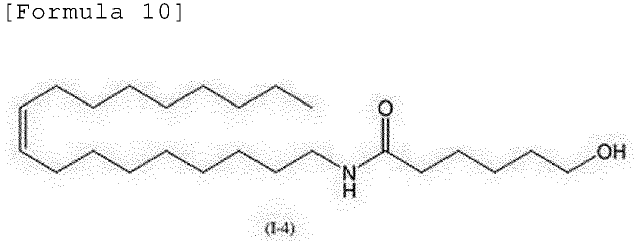

BENTONE GEL ISD V: trade name (isododecan, disteardimonium hectorite, propylene carbonate, product of Elementis Specialties Inc.)
Rheopearl KL2: trade name (dextrin palmitate, product of Chiba Flour Milling Co., Ltd.)
Rheopearl WX: trade name (dextrin palmitate/hexyldecanoate), product of Chiba Flour Milling Co., Ltd.

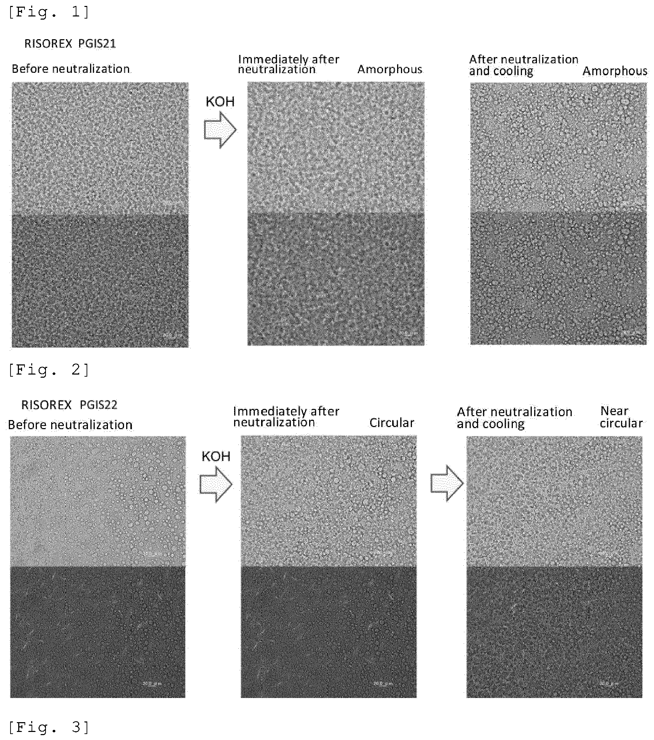

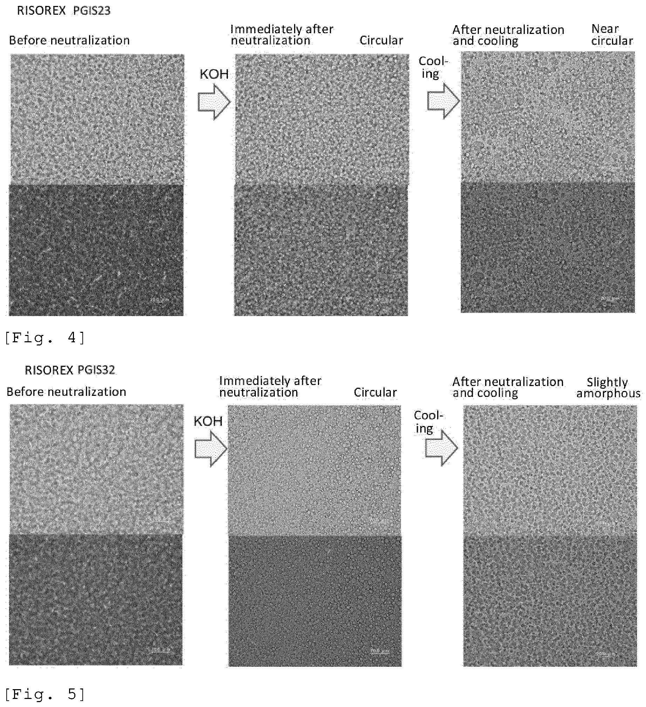

The results are shown in Table 2 below and Figs. 1 to 4.

**[Table 2]**

| | Median diameter (µm) |
|---|---|
| PGIS21 | 19.175 |
| PGIS22 | 20.469 |
| PGIS23 | 10.998 |
| PGIS32 | 15.029 |

In the W/O emulsion composition, it was confirmed that uniform particles having particularly high emulsifying power can be formed in the cases of PGIS22 and PGIS23. From this result, it is considered that a W/O emulsion composition has a particularly high emulsifying power when a polyglycerol fatty acid ester capable of forming a complex by hydrogen bonding with an amide alcohol is used.

### (2) Comparative examples of W/O emulsion composition

(2-1) The particle size and the shape of particles were confirmed using a nonionic activator containing no hydroxy group.

### [Comparative Example 1]

### EMALEX GWIS-305EX

### (Polyglyceryl triisostearate PEG-5, HLB value 3.0)

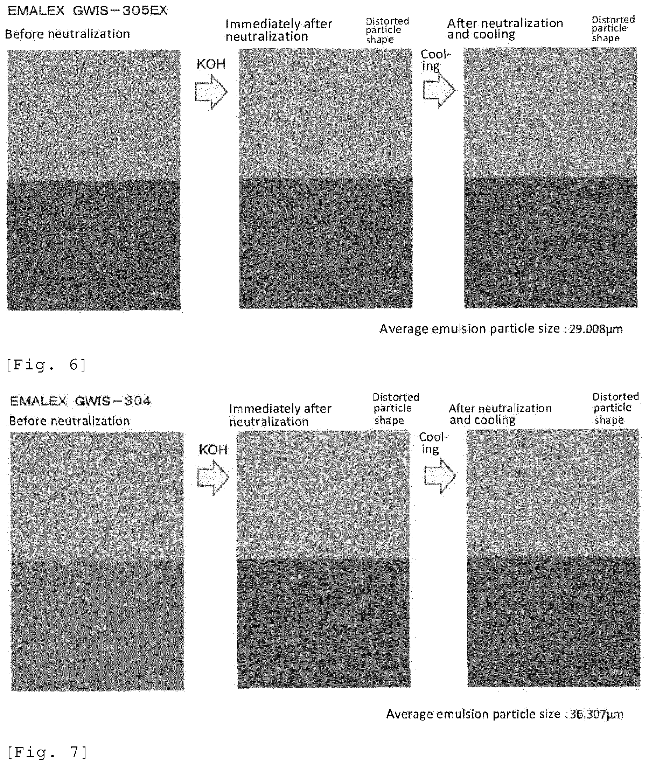

The results are shown in Fig. 5. When EMALEX GWIS-305EX was used, the particle size was not uniform and distortion was observed in the shape of the particles.

### [Comparative Example 2]

### EMALEX GWIS-304

### (Polyglyceryl triisostearate PEG-4, HLB value 2.0)

The results are shown in Fig. 6. When EMALEX GWIS-304 was used, the particle size was not uniform and distortion was observed in the shape of the particles.

### [Comparative Example 3]

### EMALEX GWS-305

### (Polyglyceryl tristearate PEG-5, HLB value 3.0)

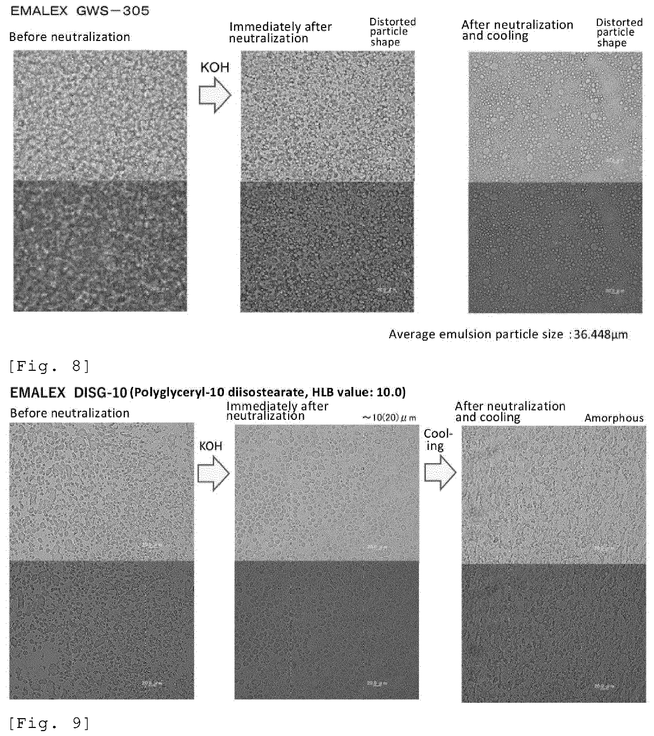

The results are shown in Fig. 7. When EMALEX GWIS-305 was used, the particle size was not uniform and distortion was observed in the shape of the particles.

(2-2) The shape of the particles was confirmed using a polyglycerol fatty acid ester having an HLB value of 10.0.

### [Comparative Example 4]

### EMALEX DISG-10

### (Polyglyceryl tristearate PEG-5, HLB value 10.0)

The results are shown in Fig. 8. When EMALEX DISG-10 was used, particles could not be observed and they were amorphous.

### [Comparative Example 5]

### EMALEX GMS-F

### (Glyceryl stearate, HLB value 5.0)

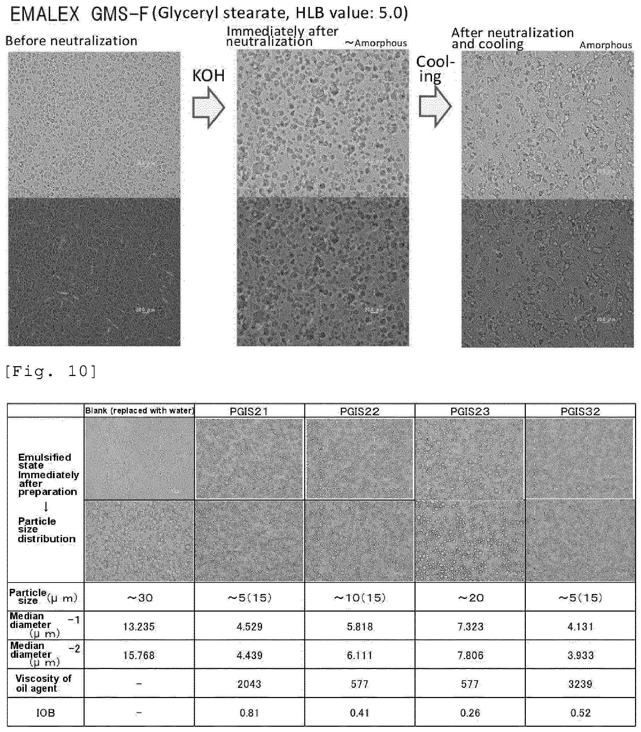

The results are shown in Fig. 9. It was confirmed that when EMALEX GMS-F was used, emulsion particles were amorphous, the emulsion particle size was large, and the emulsifying properties were not improved.

### B. Example of O/W emulsion composition

[Example 2] Using a polyglycerol fatty acid ester (Risorex), the emulsifying properties of O/W emulsion compositions were compared using particle size and median diameter.

**[Table 3]**

| | | Material name | wt% |
|---|---|---|---|
| Oil phase | 1 | Mineral oil | 12.0 |
| | 2 | Hydrogenated rapeseed oil alcohol | 3.5 |
| | 3 | Amide alcohol OLH | 2.0 |
| Aqueous phase | 4 | Polyglycerol fatty acid ester | 5.0 |
| | 5 | Glycerin | 5.0 |
| | 6 | 1,3-Butylene glycol | 3.0 |
| | 7 | 10% Potassium hydroxide solution | 1.5 |
| | 8 | Carboxyvinyl polymer | 0. 3 |
| | 9 | Purified water | 72.7 |

Here, each raw material used in the example is as follows: Polyglycerol fatty acid ester: an ester represented by formula (II), specifically as follows:
▪ RISOREX PGIS21 (polyglyceryl-2 isostearate)
▪ RISOREX PGIS22 (polyglyceryl-2 diisostearate)
▪ RISOREX PGIS23 (polyglyceryl-2 triisostearate)
▪ RISOREX PGIS32 (polyglyceryl-3 diisostearate)

Mineral oil: trade name: Hicall K-230, Kaneda Co., Ltd. Hydrogenated rapeseed oil alcohol: trade name: Alcohol No. 20-B, Kokyu Alcohol Kogyo Co, Ltd.
Amido alcohol OLH: same as that used in Example 1. Pentylene glycol: (trade name: Diol PD, Kokyu Alcohol Kogyo Co, Ltd.)
Glycerin (trade name: Triol VE, Kokyu Alcohol Kogyo Co, Ltd.) 1,3-Butanediol (trade name: Highsugarcane BG, Kokyu Alcohol Kogyo Co, Ltd.)
Carboxyvinyl polymer (trade name: Carbopol ETD2050 polymer, Lubrizol Advanced Materials, Inc.)

### Preparation method

(1) Each of oil phase and aqueous phase is uniformly dissolved at 80°C
(2) While stirring the aqueous phase with an agitator, the oil phase is added (800 rpm)
(3) After emulsification, the mixture is stirred with an agitator (2000 rpm, 3 min)
(4) Potassium hydroxide is added and the mixture is further stirred (2000 rpm, 3 min)
(5) While stirring by hand, the mixture was gradually cooled to room temperature

The results are shown in Fig. 10. In the O/W emulsion composition, the emulsifying power was confirmed to be particularly enhanced with PGIS32 and PGIS21. From this result, it is considered that the emulsifying power of O/W emulsion compositions is increased by the addition of a polyglycerol fatty acid ester, particularly a polyglycerol fatty acid ester having high viscosity and high IOB.

Hereinafter, emulsion compositions according to the formulation of the present invention were prepared. A. Examples of W/O emulsion composition

**[Table 4]**

| [Example 3] Hair treatment cream | | |
|---|---|---|
| | Ingredient name | Blending amount (%) |
| (1) | 2-Ethyl-1,3-hexanediol | 2.0 |
| (2) | Glycerin | 1.0 |
| (3) | Alkyl-modified carboxyvinyl polymer (Ingredient B) | 0.1 |
| | (Trade name: Pemulen TR-1, Lubrizol Advanced Materials, Inc.) | |
| (4) | Amide alcohol OLH of structural formula (I-4) (Ingredient A) | 3.5 |
| | (compound of structural formula (I), wherein R1 is C18, R2 is H, R3 is C5) | |
| (5) | Squalane | 35.0 |
| | (Trade name: Olive squa lane, Kokyu Alcohol Kogyo Co., Ltd.) | |
| (6) | Polyglyceryl-6 diisostearate | 3.5 |
| | (Trade name: EMALEX DISG-6, Nihon Emulsion Co., Ltd.) | |
| (7) | Methyl phenyl polysiloxane | 1.0 |
| (8) | Organically modified clay mineral premix | 4.0 |
| | (Trade name: BENTONE IHD V(isohexadecane, disteardimonium hectorite, propylene carbonate), Elementis Specialties) | |
| (9) | Isostearic acid | 3.0 |
| | (Trade name: HAIMARIC MKH (R), Kokyu Alcohol Kogyo Co., Ltd.) | |
| (10) | Dextrin palmitate | 3.0 |
| | (Trade name: Rheopearl KL2, Rheopearl TL2, Chiba Flour Milling Co, Ltd.) | |
| (11) | Keratin hydrolyzate | 1.0 |
| | (Trade name: Promois WK-H, Seiwa Kasei Co., Ltd.) | |
| (12) | Methylparaben | 0.1 |
| (13) | Octyl methoxycinnamate | 0.01 |
| (14) | Sodium hydroxide (10% aqueous solution) | 0.1 |
| (15) | Ion exchanged water | Balance |
| (16) | Fragrance | 0.1 |

### <Production method>

(4) to (10) and (13) are uniformly mixed at 80°C (oil phase). Meanwhile, (1) to (3), (11), (12) and (14) are uniformly mixed at 80°C (aqueous phase). While adding the aqueous phase to the oil phase, the resulting mixture is stirred with a disperser. Then, (14) is added and emulsified. Upon completion of the emulsification, (16) was added and cooled to room temperature to obtain a desired viscosity of 270000 mPa·s.

**[Table 5]**

| [Example 4] W/0 emulsion foundation | | |
|---|---|---|
| | Ingredient name | Blending amount (%) |
| (1) | Silicone-coated titanium oxide | 18.0 |
| (2) | Silicone-coated iron oxide (red) | 0.3 |
| (3) | Silicone-coated iron oxide (black) | 0.015 |
| (4) | Silicone-coated iron oxide (yellow) | 1.2 |
| (5) | Alkyl-modified carboxyvinyl polymer (Ingredient B) | 0.2 |
| | Trade name: Pemulen TR-2, Lubrizol Advanced Materials, Inc. | |
| (6) | Decamethylcyclopentasiloxane | 35.0 |
| (7) | Trimethylsiloxysilicate/Decamethylcyclopentasiloxane solution | 5.0 |
| | (Trade name: X-21-5250, Shin-Etsu Chemical Co., Ltd.) | |
| (8) | Amide alcohol LH of structural formula (I-3) (Ingredient A) | 3.0 |
| (9) | Polyglyceryl-10 pentaisostearate | 3.0 |
| | (Trade name: NIKKOL Decaglyn 5-ISV, Nikko Chemicals Co., Ltd.) | |
| (10) | Sodium hydroxide | 0.1 |
| (11) | Ion exchanged water | Balance |
| (12) | Disteardimonium hectorite | 5.5 |
| | (Trade name: BENTON 38V, Elementis Specialties) | |
| (13) | Isostearic acid | 5.0 |
| | (Trade name: Isostearic acid EX, Kokyu Alcohol Kogyo Co., Ltd.) | |
| (14) | Dextrin palmitate/hexyldecanoate | 3.5 |
| | (Trade name: Rheopearl WX, Chiba Flour Milling Co., Ltd.) | |
| (15) | Fragrance | Suitable amount |
| (16) | Pentylene glycol | 2.00 |
| | (Trade name: Diol PD-V, Kokyu Alcohol Kogyo Co., Ltd.) | |

### <Production method>

(1) to (4), (6) to (9), and (12) to (14) are uniformly dispersed at 80°C (oil phase). Meanwhile, an aqueous phase of (5), (11) and (16) is uniformly dissolved and mixed at 80°C, and while gradually adding the aqueous phase to the previously prepared oil phase, the resulting mixture is stirred with a disperser. Furthermore, a solution prepared with (10) and a part of (11) is added and emulsified. Upon completion of the emulsification, the mixture is cooled to room temperature to obtain a targeted W/O emulsion foundation having a viscosity of 650000 mPa·s.

**[Table 6]**

| | | |
|---|---|---|
| [Example 5] Emollient cream | | |

| | Ingredient name | Blending amount (%) |
|---|---|---|
| (1) | Hydrogenated polyisobutene | 3.0 |
| (2) | Liquid paraffin | 3.0 |
| (3) | Isostearyl neopentanoate (Trade name: Neolight 180P, Kokyu Alcohol Kogyo Co., Ltd.) | 3.0 |
| (4) | Polyglyceryl-2 diisostearate (Trade name: RISOREX PGIS22, Kokyu Alcohol Kogyo Co., Ltd.) | 4.0 |
| (5) | Decamethyl cyclopentasi loxane | 5.0 |
| (6) | (Dimethicone/phenylvinyl dimethicone)crosspolymer /diphenylsiloxy phenyl trimethicone mixture (Trade name: KSG-18A, Shin-Etsu Chemical Co., Ltd.) | 0.5 |
| (7) | Fragrance | Suitable amount |
| (8) | Amide alcohol OLB of structural formula (I-1) (Ingredient A) | 6.0 |
| (9) | Ethylparaben | 0.1 |
| (10) | Butylparaben | 0.1 |
| (11) | Tocopherol | 0.5 |
| (12) | Alkyl-modified carboxyvinyl polymer (Ingredient B) (Trade name: Pemulen TR-2, Lubrizol Advanced Materials, Inc.) | 0.15 |
| (13) | Carboxyvinyl polymer (B) ingredient (Trade name: Synthalen L, 3V Sigma USA Inc.) | 0.15 |
| (14) | Polyethylene glycol 20000 | 1.0 |
| (15) | Crataegus cuneata fruit extract | 0.1 |
| (16) | Syzygium jambos leaf extract | 0.1 |
| (17) | Aloe extract | 0.1 |
| (18) | Sanguisorba officinalis root extract | 0.1 |
| (19) | Eugenia Caryophyllus (clove) flower extract | 0.1 |
| (20) | Houttuynia cordata extract | 0.1 |
| (21) | Althaea officinalis root extract | 0.1 |
| (22) | Lithospermum officinale root extract | 0.1 |
| (23) | 1,3-Butylene glycol | 3.0 |
| (24) | Glycerin | 5.0 |
| (25) | Pentylene glycol (Trade name: Diol PD—V, Kokyu Alcohol Kogyo Co., Ltd.) | 2.0 |
| (26) | Ion exchanged water | Balance |
| (27) | Potassium hydroxide | Suitable amount |
| (28) | Organically modified clay mineral premix (Trade name: BENTONE ISD V (isododecane, disteardimonium hectorite, propylene carbonate), Elementis Specialties) | 4.0 |
| (29) | Dextrin palmitate/ethylhexanoate (Trade name: Rheopear I TT2, Chiba Flour Milling Co., Ltd.) | 3.0 |

**[Table 7]**

| | | |
|---|---|---|
| [Example 6] Whitening cream | | |

| | Ingredient name | Blending amount (%) |
|---|---|---|
| (1) | Oleic acid | 3.5 |
| (2) | Isostearic acid (Trade name: Isostearic acid EX, Kokyu Alcohol Kogyo Co., Ltd.) | 0.5 |
| (3) | Squalane (Trade name: Olive squa lane, Kokyu Alcohol Kogyo Co., Ltd.) | 25.0 |
| (4) | Polyglyceryl―2 triisostearate (Trade name: Risorex PGIS32, Kokyu Alcohol Kogyo Co., Ltd.) | 3.0 |
| (5) | Hexyl laurate (Trade name: KAK HL, Kokyu Alcohol Kogyo Co., Ltd.) | 2.0 |
| (6) | Amide alcohol LH of structural formula (I-3) (Ingredient A) | 7.0 |
| (7) | Fragrance | 0.1 |
| (8) | (Vinyldimethicone /lauryldimethicone)crosspolymer/isododecane mixture (Trade name: KSG-42, Shin-Etsu Chemical Co., Ltd.) | 0.5 |
| (9) | Tranexamic acid | 2.0 |
| (10) | Alkyl-modified carboxyvinyl polymer (Ingredient B) (Trade name: Pemulen TR-2, Lubrizol Advanced Materials, Inc.) | 0.15 |
| (11) | Methylparaben | 0.1 |
| (12) | Phenoxyethanol | 0.1 |
| (13) | Dimethicone 6cs | 5.0 |
| (14) | Glycerin (Trade name: Triol VE, Kokyu Alcohol Kogyo Co., Ltd.) | 3.0 |
| (15) | Pentylene glycol (Trade name: Diol PD-V, Kokyu Alcohol Kogyo Co., Ltd.) | 3.0 |
| (16) | Hypericumm perforatum extract | 0.1 |
| (17) | Leontopodium Alpinum extract | 0.1 |
| (18) | Royal jelly extract | 0.1 |
| (19) | Sodium ascorbyl phosphate | 0.1 |
| (20) | Ion exchanged water | Balance |
| (21) | Organically modified clay mineral premix (Trade name: BENTONE ISD V(isododecane, disteardimonium hectorite, propylene carbonate), Elementis Specialties) | 3.5 |
| (22) | Dextrin myristate (Trade name: Rheopearl MKL, Chiba Flour Milling Co., Ltd.) | 2.5 |

### <Production method>

(1) to (8), (21), and (22) are uniformly mixed at 80°C (oil phase). Meanwhile,

**[Table 8]**

| | | |
|---|---|---|
| [Example 7] Whitening beauty essence | | |

| | Ingredient name | Blending amount (%) |
|---|---|---|
| (1) | Di(phytosteryl 2-octyldodecyl)N-lauroyl-L-glutamate (Trade name: Eldew PS-203, Ajinomoto Co., Inc.) | 0.5 |
| (2) | Mineral oil | 5.0 |
| (3) | Squalane (Trade name: Olive squalane, Kokyu Alcohol Kogyo Co., Ltd.) | 15.0 |
| (4) | Isododecane | 10.0 |
| (5) | Isodecyl neopentanoate (Trade name: Neolight 100P, Kokyu Alcohol Kogyo Co., Ltd.) | 3.0 |
| (6) | Polyglyceryl-3 dicocoate (Trade name: EMALEX DCCG-3, Nihon Emulsion Co., Ltd.) | 3.0 |
| (7) | Dimethicone 5cs | 1.0 |
| (8) | Amide alcohol LB of structural formula (I-2) **(Ingredient A)** | 5.5 |
| (9) | Alkyl-modified carboxyvinyl polymer **(Ingredient B)** (Trade name: Pemulen TR-1, Lubrizol Advanced Materials, Inc.) | 0.1 |
| (10) | Carboxyvinyl polymer (Ingredient B) (Trade name: Carbopol ETD2050 Polymer, Lubrizol Advanced Materials, Inc.) | 0.05 |
| (11) | Sodium hyaluronate | 0.1 |
| (12) | Glycerin (Trade name: Triol VE, Kokyu Alcohol Kogyo Co., Ltd.) | 5.0 |
| (13) | 1,3-Butylene glycol (Trade name: Haisugarcane BG, Kokyu Alcohol Kogyo Co., Ltd.) | 3.0 |
| (14) | Ethanol | 3.0 |
| (15) | 4-Isobutyl resorcinol | 0.25 |
| (16) | Ascorbic acid glucoside | 1.0 |
| (17) | Fragrance | Suitable amount |
| (18) | Potassium hydroxide | Suitable amount |
| (19) | Sodium pyrosulfite | Suitable amount |
| (20) | Ion exchanged water | Balance |
| (21) | Pentylene glycol (Trade name: Diol PD-V, Kokyu Alcohol Kogyo Co., Ltd.) | 2.5 |
| (22) | Organically modified clay mineral premix (Trade name: BENTONE ISD V (isododecane, disteardimonium hectorite, propylene carbonate), Elementis Specialties) | 3.5 |
| (23) | Dextrin palmitate (Trade name: Rheopearl KL2, Chiba Flour Milling Co., Ltd.) | 2.5 |
| (24) | Isostearic acid (Trade name: Isostearic acid EX, Kokyu Alcohol Kogyo Co., Ltd.) | 4.0 |

### <Production method>

(1) to (8) and (22) to (24) are uniformly dissolved at 80°C (oil phase) . Meanwhile, (9) to (16) and (19) to (21) are uniformly dissolved at 80°C (aqueous phase) . The aqueous phase at 80°C is added to the oil phase at 80°C, and the resulting mixture is stirred with a disperser. An aqueous solution prepared by dissolving (18) in a part of (20) is added thereto, and the mixture is stirred with a disperser to emulsify. Upon completion of the emulsification, (17) is added and cooled to room temperature to obtain a targeted whitening beauty essence having a viscosity of 557000 mPa·s.

**[Table 9]**

| | | |
|---|---|---|
| [Example 8] W/O emulsion sunscreen | | |

| | Ingredient name | Blending amount (%) |
|---|---|---|
| (1) | Octyl p-methoxycinnamate | 3.0 |
| (2) | Glyceryl ethylhexanoate di-p-methoxycinnamate | 2.0 |
| (3) | 4-tert-buty-4' -methoxydibenzoylmethane | 2.0 |
| (4) | Tetra(octanoate/p-methoxycinnamate)pentaerythritol | 3.0 |
| (5) | Ethylhexyl isononanoate (Trade name: ES108109, Kokyu Alcohol Kogyo Co., Ltd.) | 5.0 |
| (6) | Dimethicone 20cs | 3.0 |
| (7) | Squalane (Trade name: Olive squa lane, Kokyu Alcohol Kogyo Co., Ltd.) | 20.0 |
| (8) | Amide alcohol OLB of structural formula (I-1) (Ingredient A) | 2.2 |
| (9) | Polyglyceryl-2 isostearate (Trade name: RISOREX PGIS21, Kokyu Alcohol Kogyo Co., Ltd. | 2.5 |
| (10) | Glycerin (Trade name: Triol VE, Kokyu Alcohol Kogyo Co., Ltd.) | 0. 1 4.0 |
| (11) | Ion exchanged water | Balance |
| (12) | Dipropylene glycol | 1.0 |
| (13) | Methylparaben | 0.2 |
| (14) | Alkyl-modified carboxyvinyl polymer (Ingredient B) (Trade name: Pemulen TR-2, Lubrizol Advanced Materials, Inc.) | 0.1 |
| (15) | Fragrance | 0. 1 |
| (16) | Triethanolamine | Suitable amount |
| (17) | Pentylene glycol (Trade name: Diol PD-V, Kokyu Alcohol Kogyo Co., Ltd.) | 2.5 |
| (18) | Organically modified clay mineral premix (Trade name: BENTONE ISD V (isododecane, disteardimonium hectorite, propylene carbonate), Elementis Specialties) | 3.5 |
| (19) | Dextrin palmitate (Trade name: Rheopearl KL2, Chiba Flour Milling Co., Ltd.) | 2.5 |
| (20) | Isostearic acid (Trade name: Isostearic acid EX, Kokyu Alcohol Kogyo Co., Ltd.) | 4.0 |

### <Production method>

(1) to (9), (15), (18) to (20) are uniformly dissolved at 80°C (oil phase).

Meanwhile, (10) to (14) and (17) are uniformly mixed and dissolved at 80°C (aqueous phase). This aqueous phase is slowly added to the oil phase heated to 80°C, and stirred with a disperser. Furthermore, (16) is added and emulsified. Upon completion of the emulsification, the mixture is cooled to room temperature to obtain a targeted W/O emulsion sunscreen having a viscosity of 348000 mPa·s.

It is also possible to prepare the formulations of Examples 1 to 6 by pre-adding a neutralizing agent such as potassium hydroxide, triethanolamine, sodium hydroxide, etc. to the aqueous phase.

### B. Examples of O/W emulsion composition

**[Table 10]**

| | | |
|---|---|---|
| [Example 9] Hair treatment lotion | | |

| | Ingredient name | Blending amount (%) |
|---|---|---|
| (1) | Propylene glycol | 2.0 |
| (2) | Glycerin | 1.0 |
| (3) | Alkyl-modified carboxyvinyl polymer **(Ingredient B)** (Trade name: Pemulen TR-1, Lubrizol Advanced Materials, Inc.) | 0.1 3.0 |
| (4) | Amide alcohol LB **(Ingredient A)** (Trade name: ES108109, Kokyu Alcohol Kogyo Co., Ltd.) | 0.5 |
| (5) | Methyl phenyl polysiloxane | 1.0 |
| (6) | Keratin hydrolyzate (Trade name: Promois WK-H, Seiwa Kasei Co., Ltd.) | 1.0 |
| (7) | Methylparaben | 0.1 |
| (8) | Octyl methoxycinnamate | 0.01 |
| (9) | Ethanol | 5.0 |
| (10) | Potassium hydroxide | 0.15 |
| (11) | Ion exchanged water | Balance |
| (12) | Fragrance | 0.01 |
| (13) | Polyglyceryl-2 diisostearate (Trade name: RISOREX PGIS22, Kokyu Alcohol Kogyo Co., Ltd. | 3.0 |

### <Production method>

(1) to (3), (6), (7) and (11) are heated to 80°C and uniformly dissolved (aqueous phase) . Meanwhile, (4), (5), (8) and (13) are uniformly dissolved at 80°C to obtain an oil phase. While adding the oil phase to the aqueous phase, the resulting mixture is stirred with a disperser. Then, a solution prepared with (10) and a part of (11) is added and emulsified. Upon completion of the emulsification, the mixed solution of (9) and (12) is added and cooled to room temperature to obtain a targeted hair treatment lotion having pH 6.2.

**[Table 11]**

| | | |
|---|---|---|
| [Example 10] Emulsion foundation | | |

| | Ingredient name | Blending amount (%) |
|---|---|---|
| (1) | Silicone-coated titanium oxide | 18.0 |
| (2) | Silicone-coated iron oxide (red) | 0.3 |
| (3) | Silicone-coated iron oxide (black) | 0.015 |
| (4) | Silicone-coated iron oxide (yellow) | 1.2 |
| (5) | Alkyl-modified carboxyvinyl polymer **(Ingredient B)** (Trade name: Pemulen TR-1, Lubrizol Advanced Materials, Inc.) | 3.0 |
| (6) | Decamethyl cyclopentasi loxane | 35.0 |
| (7) | Trimethylsiloxysilicate/decamethylcyclopentasiloxane solution (Trade name: X-21-5250, Shin-Etsu Chemical Co., Ltd.) | 5.0 |
| (8) | Amide alcohol of structural formula (I) **(Ingredient A)** | 3.0 |
| | (R1 is 2-ethylhexyl, R2 is H, R3 is C4) | 0.01 |
| (9) | Sodium hydroxide | 0. 1 |
| (10) | Ion exchanged water | Balance |
| (11) | Polyglyceryl-2 triisostearate (Trade name: RISOREX PGIS23, Kokyu Alcohol Kogyo Co., Ltd. | 2.5 |

### <Production method>

(1) to (4), (6), (7), (8), and (11) are uniformly dispersed at 80°C (oil phase) . Then a solution prepared with (9) and a part of (10) is added and emulsified. Upon completion of the emulsification, the resulting emulsion is cooled to room temperature to obtain a targeted emulsion foundation having pH 6.6.

**[Table 12]**

| [Example 11] Milky lotion | | |
|---|---|---|
| | Ingredient name | Blending amount (%) |
| (1) | Dimethicone 5cs | 10.0 |
| (2) | Squalane | 10.0 |
| (3) | Olefin oligomer | 6.0 |
| (4) | Polyglyceryl-2 isostearate | 5.0 |
| | (Trade name: RISOREX PGIS21, Kokyu Alcohol Kogyo Co., Ltd. | |
| (5) | Amide alcohol of structural formula (I) **(Ingredient A)** | 1.5 |
| | (R1 is octyl, R2 is H, R3 is C4) | |
| (6) | Fragrance | Suitable amount |
| (7) | Pentylene glycol | 2.0 |
| | (Trade name: Diol PD-V, Kokyu Alcohol Kogyo Co., Ltd.) | |
| (8) | 1,3-Butylene glycol | 4.0 |
| (9) | Glycerin | 6.0 |
| (10) | Carboxyvinyl polymer (Ingredient B) | 0.1 |
| | (Trade name: Carbopol 981 Polymer, Lubrizol Advanced Materials, Inc.) | |
| (11) | Alkyl-modified carboxyvinyl polymer **(Ingredient B)** | 0.1 |
| | (Trade name: Pemulen TR-2, Lubrizol Advanced Materials, Inc.) | |
| (12) | Sodium hydroxide | Suitable amount |
| (13) | Acetylated hyaluronic acid | 0.01 |
| (14) | Polymethacryloyl ethyl phosphorylcholine derivative | 0.1 |
| | (Trade name: LIPIDURE-PMB (Ph10), NOF Corporation) | |
| (15) | Equisetum arvense extract | 0.1 |
| (16) | Hamamelis virginiana (witch hazel) leaf extract | 0.1 |
| (17) | Ethanol | 5.0 |
| (18) | Phenoxyethanol | 0.3 |
| (19) | Ion exchanged water | Balance |
| (20) | Polyvinyl alcohol | 0.3 |

### <Production method>

(7) to (11) and (13) to (16) are uniformly dissolved at 80°C (aqueous phase). Meanwhile, (1) to (5) are uniformly dissolved at 80°C, added to the aqueous phase, and stirred with a homomixer at 80°C. Then, an aqueous solution of (12) dissolved in a part of (19) is added thereto, and the resulting mixture is emulsified again with a homomixer. After completion of the emulsification, the mixed solution of (6) and (7) is added and cooled to room temperature, to obtain a targeted milky lotion having pH 6.8.

**[Table 13]**

| [Example 12] Emollient cream | | |
|---|---|---|
| | Ingredient name | Blending amount (%) |
| (1) | Behenyl alcohol | 1.0 |
| (2) | Batyl alcohol | 0.5 |
| (3) | Hydrogenated polyisobutene | 3.0 |
| (4) | Liquid paraffin | 3.0 |
| (5) | Isostearyl neopentanoate | 6.0 |
| | (Trade name: NEOLIGHT 180P, Kokyu Alcohol Kogyo Co., Ltd.) | |
| (6) | Decamethyl cyclopentasi loxane | 5.0 |
| (7) | (Dimethicone/phenylvinyl dimethicone)crosspolymer /diphenylsiloxy phenyl trimethicone mixture | 0.5 |
| | (Trade name: KSG-18A, Shin-Etsu Chemical Co., Ltd.) | |
| (8) | Fragrance | Suitable amount |
| (9) | Amide alcohol of structural formula (I) **(Ingredient A)** | 2.0 |
| | (R1 is C12 alkyl, R2 is H, R3 is C4) | |
| (10) | Ethylparaben | 0.1 |
| (11) | Butylparaben | 0.1 |
| (12) | Tocopherol | 0.5 |
| (13) | Carboxyvinyl polymer **(Ingredient B)** | 0.15 |
| | (Trade name: AQUPEC 501, Sumitomo Seika Chemicals Co., Ltd.) | |
| (14) | Carboxyvinyl polymer **(Ingredient B)** | 0.15 |
| | (Trade name: Synthalen L, 3V Sigma USA Inc.) | |
| (15) | Polyethylene glycol 20000 | 1.0 |
| (16) | Crataegus cuneata fruit extract | 0.1 |
| (17) | Syzygium jambos leaf extract | 0.1 |
| (18) | Aloe extract | 0.1 |
| (19) | Sanguisorba officinalis root extract | 0.1 |
| (20) | Eugenia Caryophyllus (clove) flower extract | 0.1 |
| (21) | Houttuynia cordata extract | 0.1 |
| (22) | Althaea officinalis root extract | 0.1 |
| (23) | Lithospermum officinale root extract | 0.1 |
| (24) | 1,3-Butylene glycol | 3.0 |
| (25) | Glycerin | 5.0 |
| (26) | Ion exchanged water | Balance |
| (27) | Potassium hydroxide | Suitable amount |
| (28) | Polyglyceryl-3 diisostearate | 4.0 |
| | (Trade name: RISOREX PGIS23, Kokyu Alcohol Kogyo Co., Ltd. | |

### <Production method>

(13) to (26) and (28) are uniformly dissolved at 80°C (aqueous phase). Meanwhile, (1) to (12) are uniformly dissolved at 80°C, added to the aqueous phase, and stirred with a disperser at 80°C. Then, an aqueous solution of (27) dissolved in a part of (26) is added, and the resulting mixture is emulsified again with a disperser. After completion of the emulsification, the mixture is cooled to room temperature to obtain a targeted emollient cream having pH 7.1.

**[Table 14]**

| [Example 13] Whitening cream [ 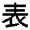 1] | | |
|---|---|---|
| | Ingredient name | Blending amount (%) |
| (1) | Palmitic acid | 2.0 |
| (2) | Cetyl alcohol | 1.5 |
| (3) | Vaseline | 0.5 |
| (4) | Squalane | 5.0 |
| | (Trade name: Olive squalane, Kokyu Alcohol Kogyo Co., Ltd.) | |
| (5) | Triehylhexanoin | 3.0 |
| | (Trade name: TOG, Kokyu Alcohol Kogyo Co., Ltd.) | |
| (6) | Hexyl laurate | 2.0 |
| | (Trade name: KAK HL, Kokyu Alcohol Kogyo Co., Ltd.) | |
| (7) | Amide alcohol of structural formula (I) **(Ingredient A)** | 2.5 |
| | (R1 is myristyl, R2 is H, R3 is C4 alkyl) | |
| (8) | Fragrance | 0. 1 |
| (9) | (Vinyldimethicone/lauryldimethicone)crosspolymer/isodode cane mixture | 0.5 |
| | (Trade name: KSG-42, Shin-Etsu Chemical Co., Ltd.) | |
| (10) | Tranexamic acid | 2.0 |
| (11) | Carboxyvinyl polymer **(Ingredient B)** | 0.5 |
| | (Trade name: Carbopol 980 Polymer, Lubrizol Advanced Materials, Inc.) | |
| (12) | Methylparaben | 0.1 |
| (13) | Phenoxyethanol | 0.1 |
| (14) | Dimethicone 6cs | 5.0 |
| (15) | Glycerin | 3.0 |
| (16) | Hypericumm perforatum extract | 0.1 |
| (17) | Leontopodium Alpinum extract | 0.1 |
| (18) | Royal jelly extract | 0.1 |
| (19) | Ion exchanged water | Balance |
| (20) | Polyglyceryl-2 oleate | 3.0 |
| | (Trade name: S Face, Sakamoto Yakuhin Kogyo Co., Ltd.) | |

### <Production method>

(10) to (19) are uniformly heated at 80°C (aqueous phase) . Then, the oil phase of (1) to (9) and (20) is uniformly dissolved at 80°C. The oil phase heated to 80°C is added to the aqueous phase heated to 80°C, and the resulting mixture is stirred with a homomixer to emulsify. Upon completion of the emulsification, the mixture is cooled to room temperature to obtain a targeted whitening cream having pH 6.7.

**[Table 15]**

| [Example 14] Whitening beauty essence | | |
|---|---|---|
| | Ingredient name | Blending amount (%) |
| (1) | Di(phytosteryl 2-octyldodecyl)N-lauroyl-L-glutamate | 0.5 |
| | (Trade name: Eldew PS-203, Ajinomoto Co., Inc.) | |
| (2) | Isostearyl neopentanoate | 3.0 |
| | (Trade name: NEOLIGHT 100P, Kokyu Alcohol Kogyo Co., Ltd.) | |
| (3) | Dimethicone 5cs | 1.0 |
| (4) | Amide alcohol of structural formula (I) **(Ingredient A)** | 4.0 |
| | (R1 is 2-ethylhexyl, R2 is H, R3 is C4) | |
| (5) | Alkyl-modified carboxyvinyl polymer **(Ingredient B)** | 0.1 |
| | (Trade name: Pemulen TR-1, Lubrizol Advanced Materials, Inc.) | |
| (6) | Carboxyvinyl polymer **(Ingredient B)** | 0.3 |
| | (Trade name: Carbopol ETD2050 Polymer, Lubrizol Advanced Materials, Inc.) | |
| (7) | Sodium hyaluronate | 0.1 |
| (8) | Glycerin | 5.0 |
| (9) | 1,3-Butylene glycol | 3.0 |
| (10) | Ethanol | 3.0 |
| (11) | 4-Isobutyl resorcinol | 0.25 |
| (12) | Ascorbic acid glucoside | 1.0 |
| (13) | Fragrance | Suitable amount |
| (14) | Potassium hydroxide | Suitable amount |
| (15) | Sodium pyrosulfite | Suitable amount |
| (16) | Ion exchanged water | Balance |
| (17) | Polyglyceryl-10 pentastearate | 2.5 |
| | (Trade name: NIKKOL Decaglyn 5-ISV, Nikko Chemicals Co., Ltd.) | |

### <Production method>

(5) to (9), (11), (12), (15), (16) and (17) are uniformly dissolved at 80°C (aqueous phase) . Then, the oil phase of (1) to (5) is uniformly dissolved at 80°C. The oil phase at 80°C is added to the aqueous phase at 80°C, and the resulting mixture is stirred with a disperser. An aqueous solution prepared by dissolving (14) in a part of (16) is added thereto, and the mixture is stirred with a disperser to emulsify. Upon completion of the emulsification, the mixed solution of (13) and (10) is added and cooled to room temperature, to obtain a targeted whitening beauty essence having pH 7.1.

**[Table 16]**

| [Example 15] Oil-in-water emulsion sunscreen | | |
|---|---|---|
| | Ingredient name | Blending amount (%) |
| (1) | Octyl p-methoxycinnamate | 6.0 |
| (2) | Glyceryl octanoate di-p-methoxycinnamate | 2.0 |
| (3) | 4-tert-buty-4'-methoxybenzoylmethane | 2.0 |
| (4) | Tetra(octanoate/p-methoxycinnamate) pentaerythritol | 3.0 |
| (5) | Ethylhexyl isononanoate | 12.0 |
| | (Trade name: ES108109, Kokyu Alcohol Kogyo Co., Ltd.) | |
| (6) | Dimethicone 20cs | 3.0 |
| (7) | Squalane | 3.0 |
| | (Trade name: Olive squalane, Kokyu Alcohol Kogyo Co., Ltd.) | |
| (8) | Amide alcohol of structural formula (I) **(Ingredient A)** | 2.2 |
| | (R1 is 2-butyl, R2 is H, R3 is C4) | |
| (9) | Microcrystalline wax | 0.1 |
| (10) | Ion exchanged water | Balance |
| (11) | Dipropylene glycol | 5.0 |
| (12) | Methylparaben | 0.2 |
| (13) | Carboxyvinyl polymer **(Ingredient B)** | 0.3 |
| | (Trade name: Carbopol 981 Polymer, Lubrizol Advanced Materials, Inc.) | |
| (14) | Fragrance | 0.1 |
| (15) | Triethanolamine | Suitable amount |
| (16) | Polyglyceryl-2 triisostearate | 3.0 |
| | (Trade name: RISOREX PGIS23, Kokyu Alcohol Kogyo Co., Ltd. | |

### <Production method>

(10) to (13) are uniformly dissolved at 80°C (aqueous phase). Then, the oil phase of (1) to (9), (14) and (16) is uniformly dissolved at 80°C. The oil phase at 80°C is added to the aqueous phase heated to 80°C, and the resulting mixture is stirred with a disperser. An aqueous solution prepared by dissolving (15) in a part of (10) is added thereto, and the mixture is again stirred with a disperser to emulsify. Upon completion of the emulsification, the mixture is cooled to room temperature to obtain a targeted oil-in-water emulsion sunscreen having pH 6.5.

## Claims

1. An emulsion composition comprising a carboxy group-containing polymer, an amide alcohol represented by formula (I): 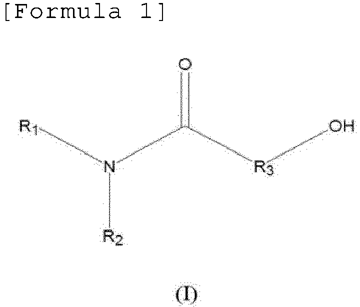 wherein
R₁ is a C6-C22 hydrocarbon group,
R₂ is H, or a C6-C22 hydrocarbon group,
R₃ is a linear or branched C2-C21 hydrocarbon group,
and a polyglycerol fatty acid ester having one or more hydroxy groups.

2. The emulsion composition according to claim 1, wherein the carboxy group-containing polymer has a weight average molecular weight of 500,000 to 3,000,000 and a carboxy group content of 50 to 70%.

3. The emulsion composition according to claim 1 or 2, wherein the polyglycerol fatty acid ester having a hydroxy group is represented by formula (II): 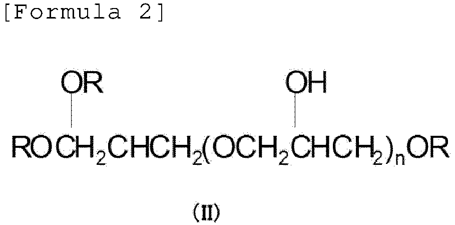 or formula (III): 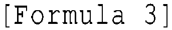 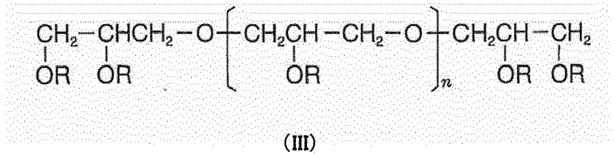 wherein R each independently represents a fatty acid residue or H,
n is an integer of 2 to 10.

4. The emulsion composition according to any one of claims 1 to 3, wherein the polyglycerol fatty acid ester having a hydroxy group has an HLB of 2 to 8.

5. The emulsion composition according to any one of claims 1 to 4, further comprising a higher fatty acid.

6. The emulsion composition according to any one of claims 1 to 5, which is an O/W type.

7. The emulsion composition according to any one of claims 1 to 5, which is a W/O type.

8. A complex formed by binding a carboxy group-containing polymer, and an amide alcohol represented by formula (I): 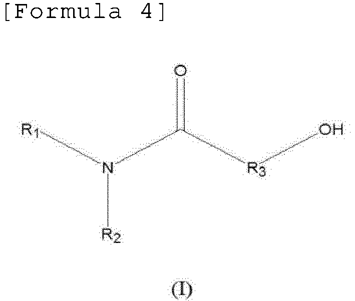 wherein
R₁ is a C6-C22 hydrocarbon group,
R₂ is H, or a C6-C22 hydrocarbon group,
R₃ is a linear or branched C2-C21 hydrocarbon group;
a polyglycerol fatty acid ester having a hydroxy group, and
a higher fatty acid.

9. The complex according to claim 8, which is used for emulsification.
